# EUROPEAN PATENT SPECIFICATION

(11) **EP 0 570 411 B1**
(45) Date of publication and mention of the grant of the patent: **04.09.1996**
(21) Application number: 92903627.5
(22) Date of filing: 04.02.1992
(51) Int. Cl.: C07C 31/125, C07C 29/16, C07C 69/80, C08K 5/10

(54) **ALCOHOL PRODUCTION**
HERSTELLUNG VON ALKOHOLEN
PRODUCTION D'ALCOOL

(30) Priority: 06.02.1991 GB 9102513
(43) Date of publication of application: 24.11.1993
(73) Proprietor: EXXON CHEMICAL PATENTS INC., Florham Park New Jersey 07932 (US)
(72) Inventor: HAMARD, Jacques, Marie, Michel, B-1330 Rixensart (BE)
(74) Representative: Bawden, Peter Charles
(86) International application number: EP9200245
(87) International publication number: WO9213818

(56) References cited:
- EP-A- 0 237 175
- EP-A- 0 278 407
- GB-A- 477 128
- GB-A- 1 531 660
- US-A- 4 123 471
- US-A- 4 229 586
- US-A- 4 291 127
- Patent Abstracts of Japan, unexamined applications, C section, vol. 3, no. 109, September 12, 1979, The Patent Office Japanese Go- vernment, Kokai-no. 54-88 206

## Description

This invention relates to the manufacture of alcohols and is concerned more especially with the oxonation of octenes obtained by dimerizing butenes over solid phosphoric acid catalyst. The invention also relates to the manufacture of nonyl alcohols and esters thereof, and the use of the esters as plasticizers.

It has long been known - see for example "Advances in Catalysis and Related Subjects", Volume 8, pp 219 to 238 (Edwin K. Jones), Academic Press Inc., 1956 - to convert butenes optionally mixed with propene, using a phosphoric acid catalyst, to octenes and heptenes for use, usually after hydrogenation and addition of tetraethyl lead, in gasoline. Although other materials have been used for catalysing polymerization, or oligomerization, reactions of this type, solid phosphoric acid catalysts are predominantly employed because of their tolerance to impurities that poison other catalysts, for example sulphur, acetylenic hydrocarbons, diolefins, nitrogen, oxygen-containing compounds, and water, the necessity for the removal of which makes the use of other catalysts economically unattractive. Solid phosphoric acid catalysts are accordingly used in the present invention.

In British Specification No. 477128 it is pointed out that the severity of polymerization conditions, as measured by reaction time and temperature, required for reaction increases progressively through the series isobutene, n-butenes and propene (although the Jones article cited above mentions that in the presence of butenes, propene is more reactive than the butenes), and that in the case of isobutene the principal product is a compound that on hydrogenation yields 2,2,4-trimethyl pentane, the standard by which octane numbers of gasolines are measured. The octane numbers of the polymerization products of n-butenes are lower.

In U.S. Patent No. 3309421, similar observations are made and a distinction is made between 1- and 2-butenes, 2-butene requiring higher temperatures and pressures and lower space rates than 1-butene to effect a given conversion. In order to provide gasoline of maximum octane number from a given feedstock, polymerization is effected under conditions that so far as possible minimize reaction of 2-butene relative to 1-butene, unreacted 2-butene being distilled from the reaction product stream and used as feedstock for alkylation, while the polymerizate is hydrogenated to form a gasoline component with the maximum proportion of triply branched material available from the starting material under consideration.

Among other references describing similar reaction conditions and the controls necessary to obtain a desired reaction product may be mentioned U.S. Patent No. 2695326, which describes, in Column 3, Table II, that at a pressure of 1000 psi g (70 bar), a feed rate of 0.32 U.S. gal/hr/lb of catalyst (2.67 dm³/hr/kg) and reaction temperature of 450°F (about 232°C), a feed comprising in mole per cent 21.3% propene, 17.3% n-butenes, 8.9% isobutene, diluted with 5.7% propane and 46.2% butane, yielded a product comprising 28.7% C₇ olefins and 55.4% C₈ to C₁₁ olefins.

Although much of the published prior art describes gasoline, alkylarylsulphonate detergents, and alkylbenzenes as end uses of the polymerization products, they may also be used as feedstock to the oxo process, the resulting aldehydes being oxidized to acids or hydrogenated to form alcohols, which latter are in turn esterified to form plasticizers, for example dioctyl and dinonyl phthalates.

It has now been found that dinonyl phthalate plasticizers imparting improved low temperature properties to polymers may be produced by a process of the type described above if the nonyl radical is derived from an octene produced from a chosen butene feedstock under chosen reaction conditions.

The present invention accordingly provides a method of oxonating an olefin obtained by oligomerising in the presence of solid phosphoric acid catalyst an olefinic feedstock, comprising butene and optionally propene and containing a molar proportion of butene, based on the total olefin content of at least 50%, and the butene containing at most 55%, advantageously at most 25%, and preferably at most 5% of isobutene, wherein the reactor is maintained within the range of from 200°C to 235°C and advantageously at least 210°C, preferably at least 220°C and the space velocity is at most 0.5 USG/h/lb of catalyst (at most about 4.1 dm³/h/kg of catalyst) and hydrogenating the product of oxonation. The reactor temperature is measured at the reactor outlet. The proportion by weight of total olefins converted is at least 0.90, advantageously 0.95, and preferably at least 0.97, the product of the temperature and the proportion by weight of total olefins converted advantageously being at least 185, more advantageously at least 200, and preferably at least 210. This product is referred to subsequently herein as "severity".

Advantageously, the pressure within the reactor is within the range of 500 to 1200 P.S.I. (about 3.45 to 8.27 MPa) and space velocity is preferably in the range of 0.1 to 0.4 USG/h/lb (about 0.8 to 3.3 dm³/h/Kg).

There is a close, inverse, correlation between plasticizer qualities, especially in relation to the low temperature properties of plasticized poly(vinyl chloride) and the proportion of tribranched molecules in the octene produced by the dimerization process, and accordingly the process is carried out in a manner such as to minimize the proportion of tribranched octenes in the product and, in particular, to maintain the tribranched octenes at a level of at most 20%, advantageously 15%, more advantageously 12%, and preferably 10%, of the total octene in the product.

Subject to the requirement for low tribranched octene levels, the oligomerization reaction is carried out within the range of reaction conditions normally employed in olefin oligomerization

The reaction conditions to obtain octenes with the desired maximum tribranched component will depend primarily on the proportion of butene in the olefin component of the feed, and the proportion of iso-butene in the butene component. It may, however, be generally stated that
(a) the higher the proportion of butenes in the olefin the higher the required severity
(b) the higher the proportion of iso-butene in the total butene, the higher the required severity
(c) as the catalyst loses activity as the reaction proceeds, to maintain the required severity it is necessary to reduce space velocity and/or increase temperature, up to the maximum temperature (235°C) specified above.

The proportion of total olefins produced represented by all isomers of octene (selectivity to octene) is reduced with an increase in severity.

The octene quality is improved both with higher olefin conversion and with temperature and these effects become more marked as the proportion of butene in the feed is increased.

As oligomerization catalyst there is used a solid phosphoric acid catalyst, comprising a phosphoric acid, e.g., orthopyro-, meta- or poly-phosphoric acid, on a solid carrier. The carrier may, for example, be a synthetic or natural porous silica or other oxide-containing material, e.g., keiselguhr, kaolin, infusorial earth, diatomaceous earth, activated clay, a zeolite, or an oxide of aluminium, zirconium, titanium or thorium. The acid is blended with the carrier to form a paste, and the paste may then be calcined and the resulting mass crushed or the paste may be extruded and pelletized and then calcined to yield uniform catalyst particles.

The catalyst may contain other components, for example, mineral talc, fullers earth, and various metals or their oxides or phosphates or pyrophosphates, e.g., nickel, copper, cobalt, zinc, manganese and, especially, iron, to modify its activity or physical properties, e.g., thermal conductivity, strength and attrition resistance.

The solid phosphoric acid catalyst advantageously contains 50 to 90% by weight of phosphoric acid.

As indicated above, the activity of the catalyst gradually falls as the reaction proceeds, and under given conditions the proportion of olefins converted accordingly falls. In order to maintain a more uniform oligomer output, a number of reactors in parallel may be provided, the catalyst in the various reactors being of different ages, and hence activity. The feedstock is divided between the reactors and the reaction products combined. The reactors may be shut down in rotation for replacemen of spent catalyst. In such an arrangement the reaction conditions in different reactors may differ. A blend of different catalysts may be employed in each reactor, if desired. Reactors with catalysts at three different levels of activity may conveniently be employed.

During operation, the oligomerization catalyst is advantageously kept hydrated, which reduces the rate of loss of activity. This may be conveniently accomplished by incorporating small quantities of water in the olefin feedstock, e.g., from 300 to 5000 molar parts per million.

Advantageously, the feedstock also contains paraffins, preferably butanes, which being unreactive act as diluents and reaction, and hence temperature, control agents, the oligomerization reaction being exothermic.

The method of the invention is applicable to diene-containing feedstocks, especially those containing up to 5000 ppm diene.

In a further embodiment, an olefin feedstock containing propene and butene is divided, as by distillation, into a relatively propene-rich stream and a relatively butene-rich stream, the butene-rich stream is divided, for example, by a conventional isobutene extraction process, into a relatively isobutene-rich stream and a relatively n-butene rich stream, and the last-mentioned stream subjected to oligomerization as defined above. By this procedure, in which propene and butenes are separated, it is possible to maximize production of desired nonenes and octenes from a mixed C₃/C₄ feedstock and maximize the use of the resulting isobutene.

Further, by maintaining a reduced level of isobutene in the feed, the oligomerization catalyst life may be prolonged.

It will be understood that the octene is a mixture of isomers, as will be the downstream products of the invention.

The product resulting from oligomerization is fractionated, if required, and the fraction containing the octene subjected to the oxo process, hydrogenation and optionally esterification to yield a plasticizer, e.g., dinonyl phthalate. These are carried out by methods known per se and will not be described in detail here.

The alcohol produced by oxonation of the octene and hydrogenation of the resulting aldehyde can be used to produce an ester of the resulting alcohol, more especially a phthalate ester of nonyl alcohol.

A polymer composition, comprising a polymer, more especially a vinyl chloride homo or copolymer, and the ester, more especially a phthalate ester can be obtained.

The following Examples illustrate the invention:

### Example 1

Using a solid phosphoric acid catalyst from U.O.P. Inc., a feedstock having the following composition was polymerized under the conditions given.

| Feedstock Composition | |
|---|---|
| Component | %, by volume |
| propylene | 0.2 |
| 1-butene | 22.8 |
| isobutene | 1.6 |
| 2-butene | 25.4 |
| saturates | 50.0 |

| Reaction Conditions | | |
|---|---|---|
| Reactor Temperature, | inlet, °C | 207 |
| | outlet, °C | 232 |
| Pressure, MPa | | 6.9 |
| Space Velocity, dm³/h/kg | | 2.17 |
| Butene Conversion, % | | 99.9 |

The olefin product was as follows:

| Carbons | 5-6 | 7 | 8 | 9 | 10 | 11 | 12 | 13+ |
|---|---|---|---|---|---|---|---|---|
| wt% | 2.8 | 4.7 | 40.2 | 12.4 | 6.2 | 6.8 | 20.1 | 6.8 |

The percentage composition of the octene was as follows:

| | |
|---|---|
| Linear | 1.7 |
| One branch | 24.4 |
| Two branches | 63.4 |
| Three branches | 10.5 |

The product was distilled, and a fraction boiling in the range 104° to 135°C was obtained, representing a 43% weight yield on butene, of the following composition:

| Carbon Number | 7 | 8 | 9 |
|---|---|---|---|
| wt% | 2.9 | 86.4 | 10.7 |

The fraction was oxonated and hydrogenated, and the resulting alcohol esterified with phthalic anhydride to produce diisononyl phthalate.

The diisononyl phthalate was used at a level of 50 parts per hundred parts of resin to produce a plasticized poly(vinyl chloride), the properties of the formulation being compared with polymer plasticized with (A) di-2-ethylhexyl phthalate and (B) a diisononyl phthalate produced from a triply branched octene.

| Plasticizer | Example 1 | Comparison | |
|---|---|---|---|
| | | A | B |
| 100% modulus, MPa | 11.4 | 10.3 | 12.8 |
| Low Temperature Flexibility, Clash Berg, °C | -27 | -29 | -17 |
| Soap Solution, % Loss | 1.1 | 5.0 | 2.0 |

Examples 2 To 9 illustrate variations in the oligomerisation reaction of the process of the invention.

### Example 2

The procedure of Example 1 was repeated, using the same feedstock and the same butene conversion rate, the reaction conditions being varied as follows:

| | | |
|---|---|---|
| Reactor Temperature, | inlet, °C | 204 |
| | outlet, °C | 216 |
| Pressure, MPa | | 4.1 |
| Space Velocity, dm³/h/kg | | 1.25 |

The percentage composition of the resulting octene was:

| | |
|---|---|
| Linear | 2.6 |
| One branch | 27.5 |
| Two branches | 63.7 |
| Three branches | 6.1 |

### Example 3

The procedure of Example 2 was repeated, with the reactor temperature varied as follows:

| | |
|---|---|
| Inlet, 192°C; | Outlet, 204°C |

The percentage composition of the resulting octene was:

| | |
|---|---|
| Linear | 1.1 |
| One branch | 20.9 |
| Two branches | 68.4 |
| Three branches | 9.6 |

### Example 4

This example illustrates the effect of varying the olefin conversion rate on the proportion of triply branched octenes in the product. An olefinic feedstock containing propene and butene (48 mole% butene; isobutene 51.78% of total butene content) was passed over a solid phosphoric acid catalyst at a pressure of 1000 p.s.i. (6.89 MPa) with a reactor outlet temperature of 420°F (216°C) at varying space velocities and olefin conversion rates. The results are shown below:

| Space Velocity dm³/h/kg | Conversion Rate % | C₈ Triple Branched % |
|---|---|---|
| 2.5 | 93.6 | 7.6 |
| 2.5 | 97.9 | 4.37 |
| 2.5 | 98.2 | 3.72 |
| 4.17 | 96.9 | 7.18 |
| 4.17 | 97.3 | 7.02 |

### Example 5

This example illustrates the effect of varying the reactor outlet temperature on the triply branched octene proportion. The feedstock contained propene and butene (46.7 mole % butene; isobutene 50.91% of total butene); pressure as in Example 4, space velocity 2.5 dm³/h/kg.

| Outlet Temperature, °C | Olefin Conversion, % | Triply Branched, % |
|---|---|---|
| 204 | 98.5 | 10.64 |
| 215 | 98.4 | 5.32 |

### Example 6

The procedure of Example 5 was repeated on a feedstock containing propene and butene (79.3 mole % butene; isobutene 51.16% of total butene), pressure as in Example 4, space velocity 2.5 dm³/h/kg.

| Outlet Temperature, °C | Olefin Conversion, % | Triply Branched, % |
|---|---|---|
| 215 | 97.3 | 20.33 |
| 232 | 97.5 | 9.15 |
| 232 | 97.7 | 8.58 |

The results show that increasing reactor temperature significantly reduces triple branching, while an increase in olefin conversion rate also improves the product.

### Example 7

This example shows the effect of changing the propene: butene ratio in the feedstock. Reaction conditions as in Example 4, space velocity 2.5 dm³/h/kg.

| C₄,% | iso-C₄ on C₄,% | Conversion % | Triple Branched % |
|---|---|---|---|
| 49.5 | 52.55 | 97.5 | 4.92 |
| 78.7 | 51.02 | 97.7 | 28.56 |

### Example 8

This example shows the effect of pressure on triple branching. The feedstock was propene and butene; butene 78.7%, isobutene on total butene 51%, reactor outlet temperature 215°C, space velocity 2.5 dm³/h/kg.

| Pressure MPa | Conversion % | Triple Branching % |
|---|---|---|
| 6.89 | 97.1 | 26.81 |
| 4.83 | 97.13 | 12.73 |

### Example 9

This example demonstrates the concept of severity, the product of reactor outlet temperature and olefin conversion proportion (fraction). An olefinic feedstock containing equimolar proportions of propene and butene, with an isobutene content 21% of total butene, was oligomerized over a solid phosphoric acid catalyst at various reactor outlet temperature and olefin conversion rates.

| Reactor Outlet, °C | Olefin Conversion | Triple Branching, % | Severity |
|---|---|---|---|
| 204 | 0.839 | 23.1 | 171 |
| 204 | 0.937 | 17.3 | 191 |
| 218 | 0.865 | 15.8 | 189 |
| 218 | 0.958 | 10.8 | 209 |
| 232 | 0.908 | 11.2 | 211 |
| 232 | 0.972 | 5.6 | 226 |

The practically linear inverse relationship between severity and triple branching, clear from the table, enables reactor temperature or olefin conversion rate to be adjusted to yield an octene product having a sufficiently low proportion of triply branched isomers.

## Claims

1. A method for the production of alcohols from olefins comprising oligomerizing in the presence of a solid phosphoric acid catalyst an olefinic feedstock comprising butene and optionally propene, and containing a molar proportion of butene, based on the total olefin content, of at least 50%, and the isobutene content of the butene being at most 55%, wherein the reactor outlet temperature is maintained within the range of from 200°C to 235°C, the space velocity is at most 4.1 dm³/h/kg of catalyst, oxonating the olefin oligomer and hydrogenating the product of oxonation.

2. A method according to claim 1 in which the proportion by weight of olefin oligomerized is at least 0.90.

3. A method as claimed in claim 1 or claim 2, wherein the isobutene content of the butene is at most 25%.

4. A method as claimed in any one of claims 1 to 3, wherein the reactor outlet temperature is at least 210°C.

5. A method as claimed in any one of claims 1 to 4, wherein the olefinic feedstock contains a saturated hydrocarbon diluent.

6. A method as claimed in any one of claims 1 to 5, wherein catalyst activity is maintained by an effective proportion of water in the feedstock.

7. A method as claimed in any one of claims 1 to 6, wherein the feedstock is obtained from a propene/butene feedstock by preparing a butene-rich fraction, and treating the butene-rich fraction to provide an n-butene rich fraction.

8. A method as claimed in any one of the preceding claims, in which the oligomer is octene.

9. The use in a process for the production of alcohols which comprises oxonating an olefin of an olefin obtained by oligomerisation of an olefinic feedstock in the presence of a solid phosphoric acid catalyst and hydrogenating the product of oxonation characterised in that the olefinic feedstock comprises butene and optionally propene, and containing a molar proportion of butene, based on the total olefin content, of at least 50%, and the isobutene content of the butene being at most 55%, wherein the reactor outlet temperature is maintained within the range of from 200°C to 235°C, the space velocity is at most 4.1 dm³/h/kg of catalyst.

10. The use according to claim 9 in which the olefin has less than 15% triple branching.

11. The use of the alcohols obtained by the process of claims 1 to 8 or the use of claims 9 and 10 in the production of plasticiser esters.

12. The use according to claim 11 in the production of phthalate esters.

## Patentansprüche

1. Verfahren zur Herstellung von Alkoholen aus Olefinen, bei dem in Gegenwart eines festen Phosphorsäurekatalysators ein olefinisches Einsatzmaterial oligomerisiert wird, das Buten und wahlweise Propen umfaßt und einen molaren Anteil Buten, bezogen auf den Gesamtolefingehalt, von mindestens 50 % enthält, wobei der Isobutengehalt des Butens höchstens 55 % beträgt, wobei die Reaktorauslaßtemperatur im Bereich von 200°C bis 235°C gehalten wird und die Raumgeschwindigkeit höchstens 4,1 dm³/h/kg Katalysator beträgt, das Olefinoligomer in einer Oxo-Synthese eingesetzt wird und das Produkt der Oxo-Synthese hydriert wird.

2. Verfahren nach Anspruch 1, bei dem der Gewichtsanteil des oligomerisierten Olefins mindestens 0,90 beträgt.

3. Verfahren nach Anspruch 1 oder Anspruch 2, bei dem der Isobutengehalt des Butens höchstens 25 % beträgt.

4. Verfahren nach einem der Ansprüche 1 bis 3, bei dem die Reaktorauslaßtemperatur mindestens 210°C beträgt.

5. Verfahren nach einem der Ansprüche 1 bis 4, bei dem das olefinische Einsatzmaterial einen gesättigten Kohlenwasserstoff als Verdünnungsmittel enthält.

6. Verfahren nach einem der Ansprüche 1 bis 5, Bei dem die Katalysatoraktivität durch einen effektiven Wasseranteil in dem Einsatzmaterial aufrechterhalten wird.

7. Verfahren nach einem der Ansprüche 1 bis 6, bei dem das Einsatzmaterial aus einem Propen/Buten-Einsatzmaterial erhalten wird, indem eine butenreiche Fraktion hergestellt wird und die butenreiche Fraktion behandelt wird, um eine Fraktion zu liefern, die reich an n-Buten ist.

8. Verfahren nach einem der vorhergehenden Ansprüche, bei dem das Oligomer Octen ist.

9. Verwendung in einem Verfahren zur Herstellung von Alkoholen, bei dem ein Olefin in einer Oxo-Synthese eingesetzt wird, das ein Olefin ist, welches durch Oligomerisierung eines olefinischen Einsatzmaterials in Gegenwart eines festen Phosphorsäurekatalysators erhalten worden ist, und das Produkt der Oxo-Synthesehydriert wird, dadurch gekennzeichnet, daß das olefinische Einsatzmaterial Buten und gegebenenfalls Propen umfaßt und einen molaren Anteil Buten, bezogen auf den Gesamtolefingehalt, von mindestens 50 % enthält, wobei der Isobutengehalt des Butens höchstens 55 % ist, wobei die Reaktorauslaßtemperatur im Bereich von 200°C bis 235°C gehalten wird und die Raumgeschwindigkeit höchstens 4,1 dm³/h/kg Katalysator beträgt.

10. Verwendung nach Anspruch 9, bei dem das Olefin weniger als 15 % Dreifachverzweigung aufweist.

11. Verwendung der nach dem Verfahren der Ansprüche 1 bis 8 erhaltenen Alkohole oder Verwendung der Ansprüche 9 und 10 zur Herstellung von Weichmacherestern.

12. Verwendung nach Anspruch 11 zur Herstellung von Phthalatestern.

## Revendications

1. Procédé de production d'alcools à partir d'oléfines, comprenant l'oligomérisation, en présence d'un catalyseur solide contenant de l'acide phosphorique, d'une charge oléfinique comprenant du butène et, à titre facultatif, du propène, et contenant une proportion molaire de butène, sur la base de la teneur totale en oléfines, d'au moins 50 %, et la teneur en isobutène du butène étant au plus égale à 55 %, dans lequel la température à la sortie du réacteur est maintenue dans la plage de 200 à 235°C, la vitesse spatiale est au plus égale à 4,1 dm³/h/kg de catalyseur, l'oxonation de l'oligomère d'oléfine et l'hydrogénation du produit d'oxonation.

2. Procédé suivant la revendication 1, dans lequel la proportion en poids d'oléfine oligomérisée est d'au moins 0,90.

3. Procédé suivant la revendication 1 ou la revendication 2, dans lequel la teneur en isobutène du butène est au plus égale à 25 %.

4. Procédé suivant l'une quelconque des revendications 1 à 3, dans lequel la température à la sortie du réacteur est d'au moins 210°C.

5. Procédé suivant l'une quelconque des revendications 1 à 4, dans lequel la charge oléfinique contient comme diluant un hydrocarbure saturé.

6. Procédé suivant l'une quelconque des revendications 1 à 5, dans lequel l'activité du catalyseur est entretenue par une proportion efficace d'eau dans la charge.

7. Procédé suivant l'une quelconque des revendications 1 à 6, dans lequel la charge est obtenue à partir d'une charge propène/butène par préparation d'une fraction riche en butène et traitement de la fraction riche en butène pour obtenir une fraction riche en n-butène.

8. Procédé suivant l'une quelconque des revendications précédentes, dans lequel l'oligomère est l'octène.

9. Utilisation, dans un procédé de production d'alcools qui comprend l'oxonation d'une oléfine, d'une oléfine obtenue par oligomérisation d'une charge oléfinique en présence d'un catalyseur solide contenant de l'acide phosphorique et hydrogénation du produit d'oxonation, caractérisée en ce que la charge oléfinique comprend du butène et, à titre facultatif, du propène, et contient une proportion molaire de butène, sur la base de la teneur totale en oléfines, d'au moins 50 %, et la teneur en isobutène du butène est au plus égale à 55 %, la température de la sortie du réacteur étant maintenue dans la plage de 200°C à 235°C et la vitesse spatiale étant au plus égale à 4,1 dm³/h/kg de catalyseur.

10. Utilisation suivant la revendication 9, dans laquelle l'oléfine a moins de 15 % de ramification triple.

11. Utilisation des alcools obtenus par le procédé suivant les revendications 1 à 8 ou utilisation suivant les revendications 9 et 10 dans la production d'esters plastifiants.

12. Utilisation suivant la revendication 11 dans la production d'esters d'acide phtalique.
